# DEMANDE DE BREVET EUROPEEN

(11) **EP 0 826 378 A2**
(43) Date de publication de la demande: **04.03.1998**
(21) Numéro de dépôt: 97202518.3
(22) Date de dépôt: 14.08.1997
(51) Int. Cl.: A61L 2/10

(54) **Enceinte éventuellement mobile de décontamination aux rayons U.V.**

(30) Priorité: 28.08.1996 FR 9610597
(71) Demandeur: Sallaz, Jean-Louis, 06300 Nice (FR)
(72) Inventeur: Sallaz, Jean-Louis, 06300 Nice (FR)
(74) Mandataire: Richebourg, Michel François

(57) **Abrégé**

La présente invention concerne une enceinte de décontamination éventuellement mobile pour matériels radiologiques, médicaux, chirurgicaux ou de laboratoires, dans le domaine médical ou tout autre domaine. Elle est particulièrement adaptée à la décontamination des cassettes de radiologie.

Cette décontamination est basée sur l'émission d'un rayonnement à action prophylactique dans le domaine de l'ultra-violet, et une orientation dans l'espace particulière des éléments à décontaminer, qui permettent de façon surprenante une décontamination très efficace en un temps très court, à la fois des faces et des arêtes.

L'orientation particulière dans l'espace peut être assurée par la présence d'un panier dans l'espace libre à l'intérieur de l'enceinte, ledit panier permettant le maintien dans la position voulue des objets à décontaminer.

## Description

La présente invention concerne le secteur technique de la décontamination de matériels radiologiques, médicaux, chirurgicaux ou de laboratoires, que ce soit dans le domaine médical ou dans tout autre domaine.

Il est en effet parfois nécessaire de décontaminer très rapidement et très efficacement un objet, parfois même lors de son transport donc dans une enceinte pouvant être mobile.

En effet, un objet doit parfois entrer en contact direct avec différents individus successivement, et donc nécessairement être contaminé par chacun. Ce problème est particulièrement important dans le domaine médical : un objet comme une cassette de radiologie entre en contact direct avec différents malades, sans compter le personnel médical ou paramédical et l'environnement, et ainsi un objet neuf ou non contaminé devient un vecteur de contamination au fur et à mesure de son utilisation donc de sa contamination dans la chaîne de travail.

Il n'est pas possible d'obtenir une décontamination optimum par simple nettoyage de la cassette. Par ailleurs, l'usage de sprays décontaminants peut être la cause d'allergies diverses.

Il était donc indispensable de résoudre ce problème, en obtenant une décontamination à la fois efficace et rapide, qui peut donc avoir lieu lors du transport de l'objet, par exemple une cassette de radiologie, d'un malade à un autre c'est-à-dire en ambulatoire.

Il n'existait pas jusque là de moyen efficace de décontamination rapide, complète et mobile pour assurer une aseptie et une antiseptie du matériel médical, chirurgical, radiologique ou autre, pendant le transport ou le stockage provisoire de ce matériel.

On connaît des méthodes de décontamination utilisant avantageusement des rayonnements dans le spectre de l'ultra-violet, qui ont une action germicide sur tout élément exposé. La demande de brevet FR-A-2 608 431 décrit un dispositif permettant une telle décontamination. Dans ce dispositif, les tubes à ultra-violet sont horizontaux, alors que les objets à décontaminer, des cassettes de radiologie, sont placés perpendiculairement par rapport aux tubes et aux parois portant les tubes. Cependant, il ne permet pas d'assurer la décontamination totale des éléments, et notamment des arêtes.

La présente invention concerne une enceinte éventuellement mobile de décontamination pour matériels radiologiques, médicaux, chirurgicaux ou de laboratoires, ladite décontamination étant basée sur l'émission d'un rayonnement à action prophylactique et une orientation dans l'espace particulière des éléments à décontaminer, qui permettent de façon surprenante une décontamination très efficace à la fois des faces et des arêtes, en un temps très court. Lorsque l'enceinte est mobile, elle peut être utilisée pour le transport des objets à décontaminer.

La présente invention permet par l'utilisation préférentielle d'un rayonnement dans le spectre de l'ultra-violet et une orientation particulière des objets à décontaminer de remédier à ce problème de décontamination.

En effet, le rayonnement ultra-violet génère une action germicide sur tout élément exposé, et provoque la production d'ozone à partir de l'air ambiant, ce qui augmente encore l'action prophylactique de l'atmosphère confinée dans l'enceinte de décontamination.

De façon surprenante, l'efficacité prophylactique d'un tel rayonnement est améliorée de façon exceptionnelle lorsque l'objet à décontaminer est orienté dans une certaine position par rapport au rayonnement.

Cette orientation dans l'espace des objets à décontaminer peut être assurée par tout support maintenant les objets dans la position voulue.

D'autres caractéristiques et avantages de l'invention seront mieux compris à la lecture de la description qui va suivre, et en se référant aux figures annexées, dans lesquelles :
- La figure 1 représente une enceinte vide de décontamination selon l'invention,
- La figure 2 est un schéma de l'emplacement des tubes à ultra-violet dans une enceinte selon l'invention,
- La figure 3 représente une vue de côté d'un panier récepteur destiné à être placé à l'intérieur de l'enceinte décrite en figure 1,
- La figure 4 représente une vue de dessus de ce panier,
- la figure 5 est un schéma du dessus d'un panier contenant les objets à décontaminer,
- La figure 6 représente une vue de face du panier représenté en figure 3,
- La figure 7 représente une vue en coupe du panier représenté en figure 3.

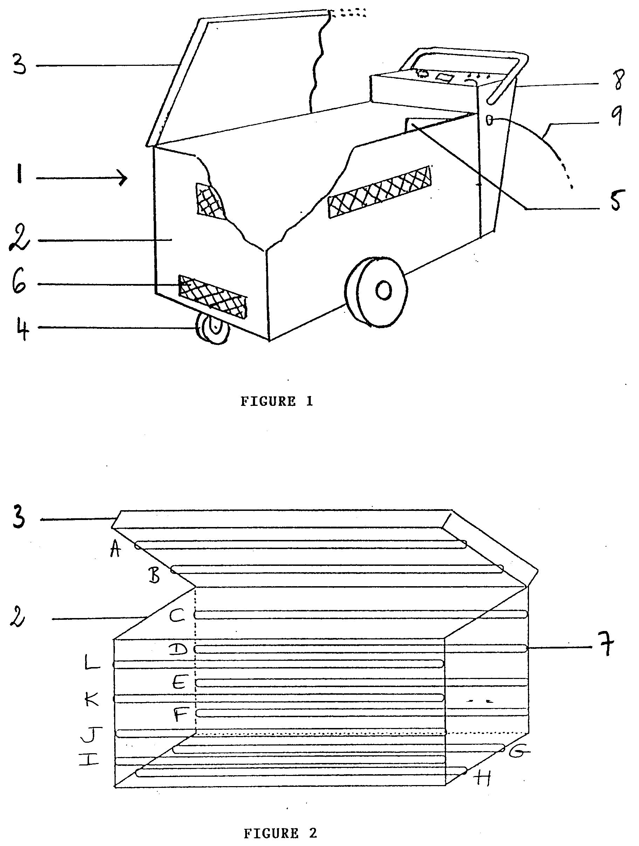

Comme on le voit sur la figure 1, l'enceinte (1) de décontamination comprend un caisson (2) muni d'un couvercle (3).

Le couvercle (3), qui permet l'accès à l'intérieur de l'enceinte, est de façon préférentiel constitué par la paroi supérieure horizontale de l'enceinte (1), afin d'assurer un accès direct à chaque objet situé dans l'enceinte. Il pourrait également être constitué par une des parois verticales.

Selon un mode de réalisation préférentiel, cette enceinte est mobile et donc munie de roues ou tout autre dispositif permettant le déplacement de ladite enceinte. Telle que représentée sur la figure 1, l'enceinte est pourvue de trois roues (4), deux d'entre elles étant situées de chaque côté de l'enceinte, la troisième étant située à l'avant. Il est bien entendu que le nombre et la position des roues peuvent varier sans que l'on sorte du cadre de l'invention.

Le caisson (2) comprend un dispositif (5) destiné à brasser l'air intérieur et à évacuer la chaleur. Ce dispositif (5) peut être un ventilateur ou tout autre dispositif remplissant la même fonction. Le caisson (2) comporte également sur ses parois verticales latérales et avant, des grilles d'aération (6) destinées à l'évacuation de l'air brassé à l'intérieur. Les faces internes des parois du caisson (2) ainsi que du couvercle (3) sont recouvertes d'un matériau réfléchissant, ou tout autre dispositif similaire permettant la réflexion des ultra-violet et donc une meilleure diffusion et une plus grande efficacité. On peut par exemple utiliser comme matériau réfléchissant de l'aluminium polyglassé dit grand-brillant.

Intégré au couvercle se trouve un "contacteur" qui permet, lors de l'ouverture ou la fermeture du couvercle, l'arrêt ou la mise en marche des tubes à UV, après mise sous tension générale.

A l'extérieur du caisson, par exemple au niveau de sa paroi arrière, se trouve un tableau de commande (8) regroupant les différentes fonctions commandées par l'opérateur. On peut notamment y faire figurer une minuterie affichant le temps nécessaire d'exposition aux UV, un interrupteur lumineux de mise sous tension-secteur, un interrupteur lumineux de mise sous tension des UV, un compteur numérique d'affichage du temps d'utilisation des UV avec remise à zéro, un ampèremètre ou tout autre moyen de contrôle de l'état des tubes UV. On trouve également à l'extérieur une poignée de conduite de l'enceinte lorsque celle-ci est mobile.

L'alimentation de l'enceinte se fait au secteur via un cordon d'alimentation (9), ou au moyen d'une batterie ou par tout autre dispositif permettant l'autonomie, avec dispositif de recharge éventuel.

L'enceinte contient les tubes à ultra-violet (7), dont la disposition sera décrite plus en détail dans la figure 2. Ces tubes sont disposés parallèlement entre eux, par l'intermédiaire de réglettes ou tout autre moyen de fixation approprié, sur les parois latérales du caisson ainsi qu'au fond du caisson et sous le couvercle.

Selon un mode de réalisation tout à tait préférentiel, on trouve sur deux des parois latérales du caisson (2) quatre tubes à UV (C à F; I à L). De plus, sur le fond du caisson (2) se trouvent également deux tubes à UV (G, H). Enfin, dans l'épaisseur du couvercle se trouvent aussi deux tubes (A, B). Il y a donc dans ce mode de réalisation préféré douze tubes à UV.

La figure 2 est un schéma de l'emplacement des tubes à UV (7) à l'intérieur du caisson (2) et du couvercle (3) d'une enceinte (1) selon l'invention.

Les objets à décontaminer sont donc placés dans l'enceinte pour subir l'action des UV. Ils sont maintenus dans la position optimum par un support, qui peut être en plastique, en fer plastifié, en aluminium, en inox, ou tout autre matériau approprié.

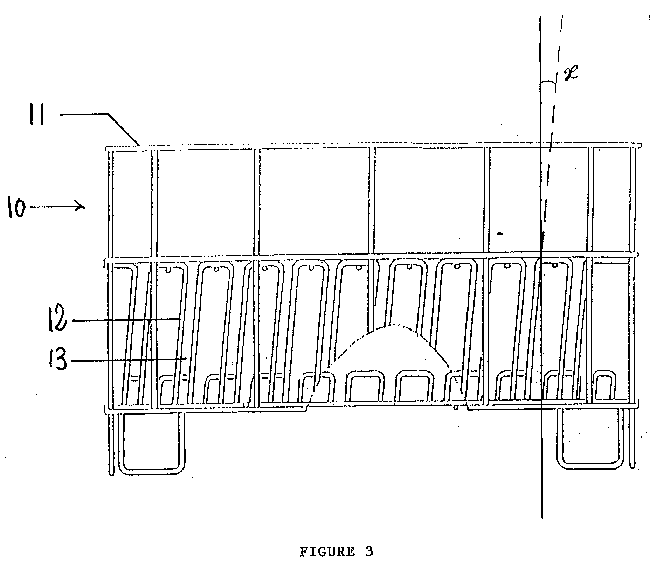

De façon préférée, ce support est constitué par un panier (10) aussi appelé panier récepteur, représenté sur les figures 3 à 7.

Le panier (10) est de préférence amovible, afin d'avoir accès à l'intérieur de l'enceinte et donc de permettre l'accès aux tubes à UV, leur remplacement éventuel et le nettoyage de l'enceinte.

Ce panier (10) vient se placer dans l'espace laissé libre à l'intérieur de l'enceinte (1). Il est constitué d'une armature (11) de dimensions appropriées pour entrer dans l'enceinte et pour contenir les objets à décontaminer, et d'un certain nombre d'arceaux (12) orientés de façon particulière. Les objets à décontaminer peuvent être placés dans l'espace libre (13) entre les arceaux, où ils sont maintenus dans la position optimale permettant la meilleure décontamination.

Cette position correspond à un léger décalage entre un côté et l'autre de l'objet à décontaminer par rapport à la paroi avant ou arrière du caisson. Ce décalage est exprimé par un angle y entre l'axe de l'objet et l'axe de propagation en ligne droite des UV, cet angle y étant au minimum de quelques degrés. De façon préférée, cet angle y est supérieur ou égal à environ 8°.

De plus, l'objet à décontaminer est placé presque verticalement, et incliné par rapport à la verticale selon un angle x de quelques degrés au minimum. De façon préférée, cet angle x est supérieur ou égal à environ 4°.

Cette orientation particulière des objets à décontaminer assure la décontamination à la fois des faces et des arêtes des objets, en évitant les rayonnements rasants inefficaces.

Ces deux angles x et y, par rapport à la verticale et par rapport à la propagation en ligne droite des UV, sont également choisis en fonction des impératifs de place. Si l'efficacité augmente quand l'angle augmente, la place occupée par les objets est également plus importante. Les valeurs les plus basses sont données pour une efficacité optimum et une perte de place aussi faible que possible.

Par ailleurs, il est indispensable que la surface de l'objet à décontaminer soumise à l'action d'un tube UV soit proche de ce tube. La distance maximale entre la source d'UV et le bord extrême de l'objet à décontaminer sera avantageusement réduite au minimum.

Dans le cas où les objets à décontaminer sont des cassettes de radiologie, les arceaux sont préférentiellement au nombre de onze, pour permettre le transport et la décontamination de dix cassettes à la fois, ce qui est le nombre préféré dans les services hospitaliers.

Dans ce cas de décontamination de 10 grandes cassettes de radiologie, l'angle y par rapport à l'axe de propagation en ligne droite des UV est de préférence compris entre environ 8° et environ 12°, de préférence encore égal à environ 10°. L'inclinaison x par rapport à la verticale est de préférence comprise entre environ 4° et environ 6°, de préférence encore égale à environ 5°. La distance maximum entre la source d'UV et l'extrémité de la surface de l'objet soumise à cette source est de préférence inférieure à environ 50 cm, de façon encore préférée inférieure à 43 cm.

Ce nombre d'objets à décontaminer n'est pas une obligation. Par exemple, il existe des grandes cassettes de radiologie (environ 36 x 43) et des petites (environ 13 x 18). Il va de soi que dans un caisson suffisamment grand pour contenir dix grandes cassettes, on peut également décontaminer vingt petites cassettes, en en plaçant deux côte à côte par intervalle.

La figure 3 représente une vue de côté d'un panier selon l'invention, pouvant contenir onze grandes cassettes de radiologie. L'angle x représente l'inclinaison des arceaux par rapport à la verticale, et donc l'inclinaison des objets placés dans l'espace libre (13) entre les arceaux par rapport à la verticale.

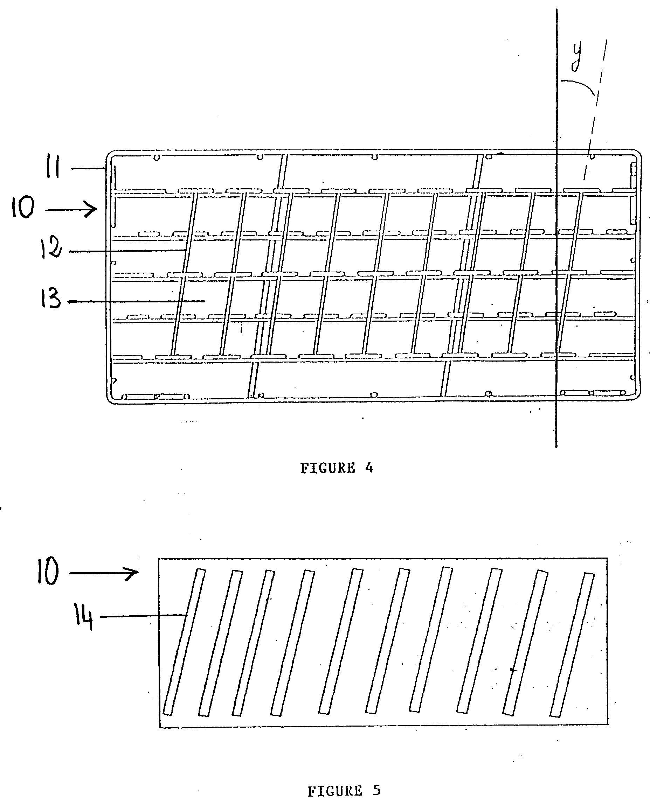

La figure 4 représente une vue de dessus de ce panier (10), permettant de visualiser le décalage entre les côtés des arceaux (12) par rapport aux parois avant et arrière du caisson, c'est-à-dire l'angle y entre l'axe de l'arceau et l'axe de propagation en ligne droite des UV et donc l'angle y entre l'axe de l'objet placé dans l'espace libre (13) entre les arceaux et l'axe de propagation en ligne droite des UV.

La figure 5 schématise la disposition de dix cassettes (14) rangées dans un panier (10) selon l'invention.

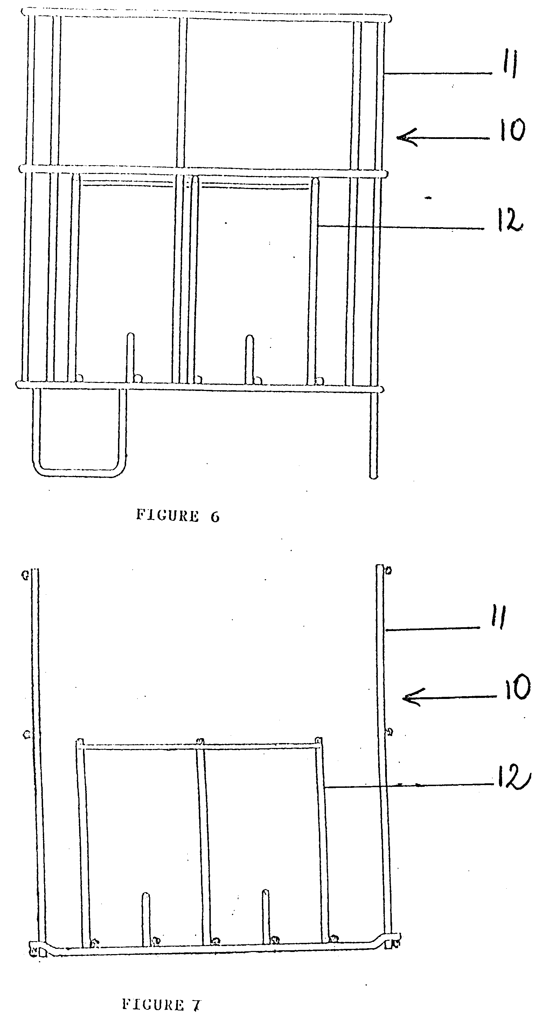

Les figures 6 et 7 sont des vues de lace et en coupe respectivement du panier (10). Y figurent l'armature (11) ainsi que les arceaux (12).

L'orientation particulière des objets dans l'espace, et donc par rapport aux tubes à UV permet d'obtenir une exposition optimale aux UV, en évitant les faisceaux rasants moins efficaces et en permettant la décontamination de l'objet dans son entier, que se soit ses faces ou ses arêtes. La décontamination obtenue est donc particulièrement efficace en un temps très court.

Cette efficacité de décontamination est illustrée par les exemples suivants.

### Exemple 1

Des essais sont effectués en laboratoire, pour évaluer l'efficacité de décontamination du dispositif selon l'invention vis à vis de certains germes représentatifs de la diversité de la flore microbienne pathogène impliquée dans les infections nosocomiales d'origine bactérienne et fongique.

On utilise un caisson de dimensions 1020 x 580 x 740 équipé intérieurement de 12 tubes UV d'une puissance de 30 watts chacun. Les parois internes du caisson sont recouvertes d'une surface réfléchissante. Les objets décontaminés sont des cassettes de radiologie de dimensions 385 x 455 x 10, disposées dans le panier selon un angle y d'environ 10° par rapport à l'axe de propagation en ligne droite des UV, et selon un angle x d'environ 5° par rapport à la verticale.

Les souches utilisées sont les suivantes :

| | |
|---|---|
| Ca = Candida albicans | Référence CIP 1180.79 |
| An = Aspergillus niger | 1431.83 |
| Sa = Staphylococcus aureus | 53.154 |
| Pa = Pseudomonas aeruginosa | A22 |

Principe :
Des porte-germes sont découpés dans des cassettes de radiographies de manière à ce que le support soit représentatif du matériau destiné à être décontaminé. Ces porte-germes sont ensemencés, puis fixés sur les cassettes de radiologie elles-même placées ensuite dans le dispositif, et soumis à l'irradiation aux UV dans le dispositif pendant 5 minutes. Après incubation à une température et un temps appropriés pour chaque souche, les microorganismes survivants font l'objet d'une numération. La valeur obtenue est comparée à celle obtenue pour des témoins non irradiés.

Il est démontré par des essais préalables non détaillés ici que les souches sont viables dans les conditions de l'essai et que les porte-germes n'exercent aucune activité antimicrobienne susceptible d'interférer avec l'action des UV.

Pour chaque souche, on titre la solution de travail et on détermine la quantité de microorganismes déposés sur les porte-germes. On ensemence trois porte-germes par souche, que l'on fixe ensuite sur différentes cassettes dans le dispositif.

Pour chaque souche, on réalise 3 témoins de récupération (non irradiés) ainsi que 3 essais proprement dits. Chaque résultat correspond à la moyenne des 3 mesures.

Les résultats sont donnés dans le tableau suivant :

**Tableau 1**

| **SOUCHE** | **Quantité déposée** | **Témoin (T)** | **Essai** | **Diminution/T (Efficacité)** |
|---|---|---|---|---|
| **Ca** | 6,0 10⁶ | 2,5 10⁵ soit 5,4 log₁₀ | 2,0 10³ soit 3,3 log₁₀ | 2,1 log₁₀ **> 99 %** |
| **An** | 4,5 10⁶ | 1,6 10⁵ soit 5,2 log₁₀ | 7,9 10³ soit 3,9 log₁₀ | 1,3 log10 **> 90 %** |
| **Sa** | 8,0 10⁷ | 4,0 10⁶ soit 6,6 log₁₀ | 316 soit 2,5 log_{1**0**} | 4,1 log₁₀ **> 99,99 %** |
| **Pa** | 4,0 10⁷ | 6,3 10⁵ soit 5,8 log₁₀ | 50 soit 1,7 log₁₀ | 4,1 log₁₀ **> 99,99 %** |

La comparaison des témoins récupération et des essais témoigne d'une réduction importante de la contamination des porte-germes. L'efficacité du dispositif selon l'invention est toujours très élevée : de > 90 % à > 99,99 % après seulement 5 minutes.

### Exemple 2

Le dispositif de décontamination selon l'invention est utilisé dans deux services de réanimation, pour 13 malades au total. Ces essais ont lieu in situ, c'est-à-dire dans des conditions réelles d'utilisation.

On teste la capacité de décontamination des cassettes de radiologie après radiographie au lit du malade.

On utilise un caisson de dimensions 1020 x 580 x 740 équipé intérieurement de 12 tubes UV d'une puissance de 30 watts chacun. Les parois internes du caisson sont recouvertes d'une surface réfléchissante. Les objets décontaminés sont des cassettes de radiologie de dimensions 385 x 455 x 10, disposées dans le panier selon un angle y d'environ 10° par rapport à l'axe de propagation en ligne droite des UV, et selon un angle x d'environ 5° par rapport à la verticale.

Les étapes sont les suivantes :
1. Décontamination de l'ensemble des cassettes avant le départ pour la tournée de radiographies : 5 minutes d'exposition dans l'appareil, qui sert de stockage ambulatoire pour les cassettes.
2. Pour chaque patient : on effectue une radiographie avec une cassette décontaminée, puis l'opérateur remet la cassette à son emplacement et en prend une deuxième avec une paire de gants propres pour le patient suivant. L'appareil restant sous tension, le cycle de stérilisation redémarre lorsque le couvercle est refermé.
3. Au retour, on effectue une décontamination supplémentaire de 5 minutes avant de développer les films.

Le matériel utilisé pour le contrôle bactériologique est le suivant :
- lames de surface OXOID 50391, avec neutralisants (lécithine tween 80), comportant deux faces, l'une recouverte de Plate count agar (numération des germes totaux), l'autre de gélose de Mac Conkey (germes gram négatif),
- gélose de Drigalski,
- gélose Columbia sang de mouton,
- galeries d'identification API 20 E ou/et 20 NE,
- milieu de Chapman Dnase et coagulase.

La méthode utilisée pour le contrôle bactériologique est la suivante :
Le contrôle microbiologique est effectué sur chaque cassette avant la radiographie , après la radiographie (contact avec le patient) et après un passage d'au moins 5 minutes dans le dispositif selon l'invention.
Ce contrôle est effectué à l'aide de lames de surface utilisées selon les directives du fournisseur, lesdites lames étant incubées 24 à 48 heures à 37°C.

Lecture des résultats :
La surface de chaque milieu est de 10,1 cm². On procède au dénombrement des bactéries sur chaque face et on exprime le résultat en germes/cm². Les différentes types de colonies sont réisolées sur milieu approprié puis identifiés.

Les résultats des essais figurent dans le tableau suivant :

**Tableau 2**

| **PATIENTS** | **CASSETTES** | | |
|---|---|---|---|
| | Avant radiographie | Après radiographie | Après décontamination |
| 1 | S | S | S |
| 2 | GE (1) | Sb (>100) | S |
| 3 | S | Ab (1) | S |
| 4 | S | Sb (5) | S |
| 5 | S | Sb (2) | S |
| 6 | S | Sb (3) | S |
| 7 | S | Sb (100) | S |
| 8 | S | Sb (2) | S |
| 9 | S | Sb (10) | S |
| 10 | S | S | S |
| 11 | S | S | S |
| 12 | S | Sb (10) + Pa (0,1) | S |
| 13 | GE (0,4) | S | S |
| GE = Germes de l'environnement Sb = Staphylocoque blanc Ab = Acinetobacter baumannii Pa = Pseudomonas aeruginosa S = Stérile | | | |

Résultats :
Les prélèvements effectués avant la radiographie sont tous stériles, à l'exception de ceux des patients 2 et 13. En revanche, les prélèvements effectués juste après la radiographie ont été positifs dans 9 cas sur 13 (soit 69%).
Les bactéries isolées sont du staphylocoque blanc (8 cas dont 2 avec un nombre de colonies au moins égal à 100/cm²), de l'*Acinetobacter baumanii* pour 1 cas, et du *Pseudomonas aeruginosa* pour le cas restant.
Après décontamination à l'aide du dispositif selon l'invention, les prélèvements sont tous stériles.

Cet exemple illustre l'intérêt d'une telle décontamination efficace et rapide, en ambulatoire, face aux risques de transmission de germes par l'intermédiaire des cassettes (69% positifs) dans la radiologie ambulatoire.

L'invention n'est naturellement pas limité aux exemples de réalisation décrits ci-dessus. L'homme de métier pourra adapter les éléments à chaque cas particulier.

En particulier, l'homme de métier saura envisager les variantes possibles, notamment l'adaptation des angles x et y d'inclinaison et de décalage en fonction de la taille et de la forme de l'objet à décontaminer.

## Revendications

1. Enceinte (1) de décontamination comprenant un caisson (2),un couvercle (3) et un dispositif de réception et de maintien (10) des objets à décontaminer, munie sur quatre de ces faces internes d'au moins un tube à ultra-violet (7), lesdits tubes étant parallèles entre eux, ladite enceinte recevant des objets à décontaminer, **caractérisée on ce que** les objets à décontaminer sont orientés dans une position particulière par rapport aux tubes à UV qui évite les rayonnements rasants et permet donc la décontamination totale à la fois des faces et des arêtes des objets.

2. Enceinte selon la revendication 1 **caractérisée en ce qu**'elle est mobile et munie d'un dispositif permettant son déplacement.

3. Enceinte selon la revendication 2 **caractérisée en ce qu**'elle est munie de roues.

4. Enceinte selon l'une quelconque des revendications 1 à 3 **caractérisée en ce que** chaque objet à décontaminer est positionné de façon telle que l'inclinaison par rapport à la verticale est au minimum d'environ 4° et le dégagement d'un côté de l'objet par rapport à l'axe de propagation en ligne droite des UV est d'environ 8° minimum, les tubes à UV étant horizontaux.

5. Enceinte selon l'une quelconque des revendications 1 à 4 **caractérisée en ce que** l'angle d'inclinaison par rapport à la verticale, les tubes à UV étant horizontaux, est d'environ 5°.

6. Enceinte selon l'une quelconque des revendications 1 à 5 **caractérisée en ce que** l'angle de dégagement d'un côté de l'objet par rapport à l'axe de propagation en ligne droite des UV est d'environ 10°.

7. Enceinte selon l'une quelconque des revendications 1 à 6 **caractérisée en ce que** l'enceinte est munie de 12 tubes à UV parallèles (7), répartis sur quatre parois, 4 tubes sur chacune des 2 grandes parois latérales se faisant face (C à F; I à L), 2 sur le plancher de l'enceinte (G, H) et 2 sous le couvercle (A, B).

8. Enceinte selon l'une quelconque des revendications 1 à 7 **caractérisée en ce que** le dispositif (10) permettant le maintien des objets à décontaminer dans la position voulue est un panier constitué d'une armature (11), et d'arceaux (12) orientés par rapport aux tubes à UV dans la position appropriée et entre lesquels sont placés les objets à décontaminer.

9. Enceinte selon l'un quelconque des revendications 1 à 8 **caractérisée en ce qu**'elle comprend un dispositif pour brasser l'air et évacuer la chaleur.

10. Enceinte selon l'un quelconque des revendications 1 à 9 c**aractérisée en ce qu**'elle comprend une source d'énergie autonome.
